# EUROPEAN PATENT APPLICATION

(11) **EP 1 336 406 A1**
(43) Date of publication of application: **20.08.2003**
(21) Application number: 02076344.7
(22) Date of filing: 14.02.2002
(51) Int. Cl.: A61K 31/138, A61K 31/495, A61K 31/445, A61K 31/535, A61K 31/55, A61K 31/48, A61P 25/00

(54) **Partial dopamine-D2 receptor agonist plus serotonin and/or noradrenaline inhibitory activity**

(71) Applicant: Solvay Pharmaceuticals B.V., 1381 CP Weesp (NL)
(72) Inventor: The designation of the inventor has not yet been filed
(74) Representative: Muis, Maarten

(57) **Abstract**

The invention relates to the use of a compound or a combination of compounds having partial dopamine-D₂ receptor agonistic activity and serotonin and/or noradrenaline reuptake inhibitory activity, for the preparation of a pharmaceutical composition for the treatment of psychiatric and/or neurologic disorders caused by disturbances of the major monoaminergic (dopamine, serotonin and/or nordrenaline) systems or that can be treated via manipulation of those systems.

## Description

The invention relates to the use of a compound or a combination of compounds having partial dopamine-D₂ receptor agonistic activity and serotonin and/or noradrenaline reuptake inhibitory activity, for the preparation of a pharmaceutical composition for the treatment of psychiatric and/or neurologic disorders caused by disturbances of the major monoaminergic (dopamine, serotonin and/or nordrenaline) systems or that can be treated via manipulation of those systems, said disorders selected from the group consisting of: schizophrenia and other psychotic disorders; mood disorders such as bipolar I disorders, bipolar II disorders and unipolar depressive disorders like minor depression, seasonal affective disorder, postnatal depression dysthymia and major depression; anxiety disorders including panic disorder (with or without agoraphobia), social phobia, obsessive compulsive disorder (OCD, with or without co-morbid chronic tic or schizotypal disorder), posttraumatic stress disorder and generalized anxiety disorder (GAD); substance related disorders, including substance use disorders (like dependence and abuse) and substance induced disorders (like substance withdrawal); pervasive development disorders including autistic disorder and Rett's disorder; attention deficit and disruptive behavior disorders such as attention deficit hyperactivity disorder (ADHD); impulse control disorders like pathological gambling; eating disorders like anorexia nervosa and bulimia nervosa; tic disorders like Tourette's disorder; restless legs syndrome; disorders characterized by impairment of cognition, memory and/or co-morbid psychiatric disorders and neurorehabilitation (post-traumatic brain lesions).

Dopaminergic neurones, particularly those of the nigrostriatal pathway are involved in the fine-tuning of control of movement. Degeneration of this pathway may lead to neurological disorders. However, dopamine in the brain is also part of the limbic system, including the limbic cortex, amygdala, nucleus accumbens, septum, olfactory tubercle and frontal cortex. Therefore disturbances in these systems are linked to disturbances of perception and especially of emotional behavior.

Serotonergic and noradrenergic projections regulate many behavioural and affective states that are disturbed in psychiatric disorders. Serotonin and noradrenaline reuptake inhibitors and also compounds with these two activities combined, are widely used for the treatment of depressive and anxiety disorders.

In contrast to the use of full dopamine-D₂ receptor agonists or antagonists, the use of partial dopamine-D₂ receptor agonists offers a dynamic medication that self-adjusts on a moment-to-moment basis to the endogenous state of the patient. Thus, it provides the desired flexible modulation of the dopamine system and avoidance of the many adverse effects caused either by treatment using full dopamine-D₂ receptor agonists like bromocriptine (hallucinations, nausea, vomiting, dyskinesia, orthostatic hypotension, somnolescence) or full dopamine-D₂ receptor antagonists like haloperidol (emotional blunting, dysphoria, tardive dyskinesia). Because of these many adverse effects, full agonists and antagonists have found only very limited use in the therapy of depressive and anxiety disorders.

Partial dopamine-D₂ receptor agonists not only show a flexible modulation and a favourable side-effect profile, they also have a pronounced anxiolytic profile in relevant animal models (Drugs of the Future 2001, 26(2): 128-132). Noradrenaline and/or serotonin reuptake inhibitors have a more pronounced antidepressive profile.

It has now been found that when both activities are combined in one pharmaceutical preparation, such preparations allow for a complete treatment of all disease symptoms (e.g. positive and negative symptoms of schizophrenia), and are particularly useful for the treatment of psychiatric disorders involving hypo-, hyper- or fluctuating activity of the dopaminergic system. Such preparations can also be used to treat patients suffering from mania, anxiety or depression in combination with psychotic episodes.

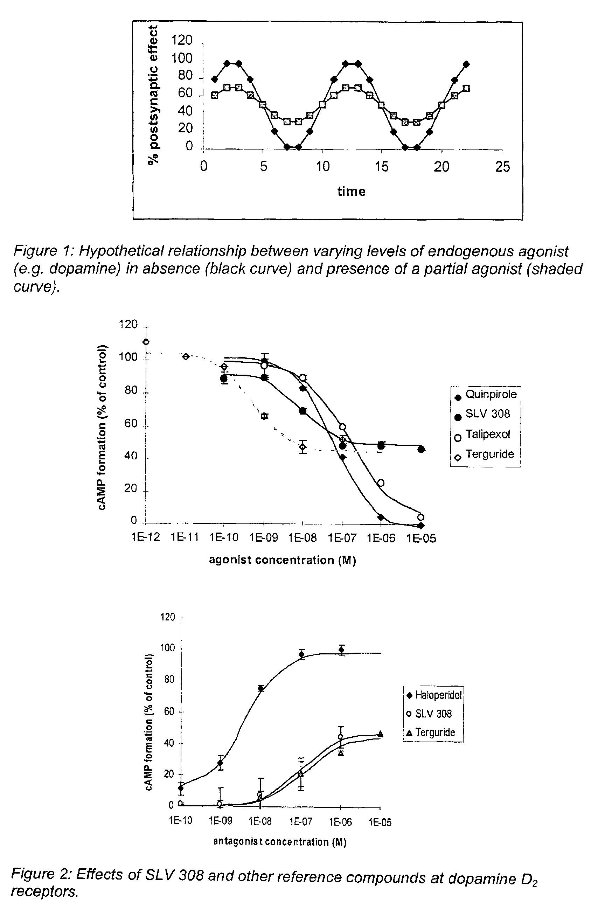

Partial dopamine-D₂ receptor agonists, according to the present invention, are compounds that - when tested in a concentration response range - achieve at least 20% but not more than 60% activation in the functional cAMP cell based assay (as described below) even in very high concentrations such as 100 times the EC₅₀-value of the compound. Compounds which give less than 20% or more than 60% activation in this functional dopamine-D₂ receptor assay are regarded as full antagonists and agonists, respectively, and are prone to cause the adverse effects associated with dopamine-D₂ receptor antagonists and agonists. Partial dopamine-D₂ receptor agonists will act as an agonist in cases when the endogenous synaptic tone of dopamine is low, or in the the presence of a full dopamine-D₂ receptor antagonist, and will act as an antagonist in cases when the endogenous synaptic tone of dopamine is high, or in the presence of a full dopamine D₂ receptor agonist. This is illustrated in Figure 1, in a graphical representation of the hypothetical relationship between varying levels of endogenous agonist (e.g. dopamine) in absence and presence of a partial agonist showing that primarily the amplitude is affected, ensuring increased tone at low ambient dopamine concentrations, and limiting peak effects at high levels.

Like full agonists, partial dopamine-D₂ receptor agonists in general are active in sensitized systems. They induce contralateral turning in rats with unilateral 6-hydroxy-dopamine (6-OHDA) lesions in the substantia nigra pars compacta. In MPTP-treated common marmosets they produce potent and long-lasting reversal of motor symptoms (Drugs of the Future 2001, 26(2): 128-132). In contrast to full agonists, however, partial dopamine-D₂ agonists are substantially less active in non-sensitized systems: they hardly reverse reserpine induced hypolocomotion in rats.

It has now been found that compounds having partial dopamine-D₂ activity and serotonin and/or noradrenaline reuptake inhibitory activity in one molecule, or pharmaceutical preparations consisting of combinations of compounds having partial dopamine-D₂ activity and serotonin and/or noradrenaline reuptake inhibitory activity, simultaneously show all three (respectively two) activities in vivo, as was demonstrated by microdialysis experimentation.

For the treatment of CNS disorders involving an overactive dopaminergic system a pharmaceutical preparation combining partial dopamine-D₂ receptor agonistic activity having low intrinsic functional activity with serotonin and/or noradrenaline reuptake inhibitory activity is recommended. In case of a disorder involving dopamine insufficiency a pharmaceutical preparation combining partial dopamine-D₂ receptor agonistic activity with high intrinsic functional activity and serotonin and/or noradrenaline reuptake activity according to the invention has considerable advantages.

Surprisingly, it has now been found that pharmaceutical preparations of one or more compounds combining an intrinsic functional dopamine activity of at least 20% and at the most 60% in combination with serotonin and/or noradrenaline reuptake activity, are useful for the treatment of all psychiatric disorders for which dynamic readjustment of the dopamine system is required.

Disorders characterized by dynamic fluctuations in dopamine neurotransmission like bipolar depression and addiction will profit in particular from the flexible adjustment of the dopamine system by the partial dopamine-D₂ receptor agonists in the pharmaceutical preparation. Combining this "dopaminergic neurotransmission stabilising" activity with serotonin and/or noradrenaline reuptake inhibitory activity will enhance antidepressive and anxiolytic efficacy.

In conclusion, the present invention demonstrates that the broad efficacy of pharmaceutical preparations, combining partial dopamine-D₂ receptor agonistic activity with serotonin and/or noradrenaline reuptake inhibitory activity in animal models predictive for antipsychotic, antidepressive and anxiolytic activity, clearly underlines the use potential of the dynamic modulation of dopamine mediated neurotransmission in combination with the antidepressive action of 5-HT and/or NA inhibitory activity for the treatment of many co-morbid psychiatric disorders.

### Examples

**Combinations** of compounds which can be used according to the invention are preparations containing *in general terms:*
(1) a partial dopamine-D₂ agonist (defined as above), a specific 5-HT reuptake inhibitor and/or a specific noradrenaline reuptake inhibitor.
(2) a partial dopamine-D₂ agonist and a compound having both 5-HT- and noradrenaline reuptake activity
(3) a partial dopamine-D₂ agonist which is also a specific 5-HT reuptake inhibitor combined with a specific noradrenaline reuptake inhibitor
(4) a partial dopamine-D2 agonist which is also a specific noradrenaline reuptake inhibitor combined with a specific 5-HT reuptake inhibitor

Specific examples of compounds which can be used in combination preparations according to the invention are (but are not restricted to) the specific serotonin reuptake inhibitors (SSRI's): alaproclate, citalopram, fluoxetine, fluvoxamine, litoxetine, nefazodone, paroxetine, sertraline, trazodone and zimelidine; the specific noradrenaline reuptake inhibitors (SNRI's): amoxapine, desipramine, maprotiline, mazindol, nisoxetine, nomifensine, nortriptiline, protriptiline, reboxetine and tomoxetine; the compounds with combined serotonin and noradrenaline reuptake inhibitory activity: chlorimipramine, duloxetine, imipramine, indatraline, milnacipran, S-33005, sibutramine and venlafaxine; and the partial dopamine-D₂ agonists: BP 897, dihydroergocristine, dihydroergotamine, preclamol ((S)-(-)-3-PPP), terguride, bifeprunox and SLV 308 (Structure (1) of examples, in which R = CH₃).

**Single compounds** which can be used according to the invention are compounds that are both partial dopamine-D₂ agonists and specific 5-HT reuptake inhibitors, for instance phenylpiperazine derivatives with the formula (1): 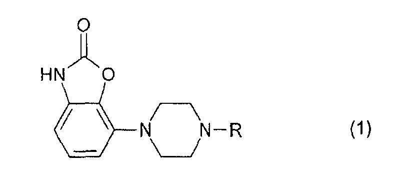 wherein R is consisting of moieties (a), (b), (c), (d) or (e) and salts thereof.

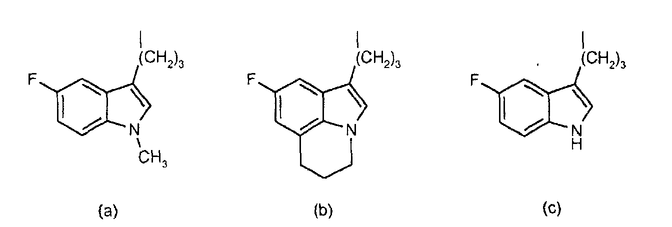

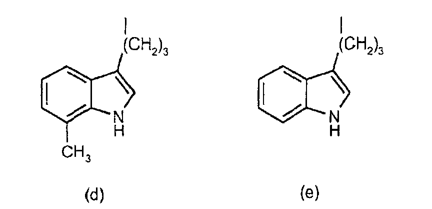

Pharmacologically acceptable acids with which the compounds of the invention can form suitable acid addition salts are for example hydrochloric acid, sulphuric acid, phosphoric acid, nitric acid, and organic acids such as citric acid, fumaric acid, maleic acid, tartaric acid, acetic acid, benzoic acid, p-toluene sulphonic acid, methanesulphonic acid and naphthalene sulphonic acid.

The compounds are their acid addition salts can be brought into forms suitable for administration by means of suitable processes using auxiliary substances such as liquid and solid carrier materials.

The examples 1a - 1e can be synthesized as described in WO 00EP08190.

### Pharmacological Testing

The *in vitro* functional activity at dopamine-D₂ receptors, including the intrinsic activity (ε) of the compounds which can be used according to the invention as well as relevant reference compounds was measured by their ability to inhibit forskolin-induced [³H]-cAMP accumulation. Serotonin and noradrenaline reuptake inhibitory activity was measured in rat brain synaptosomes. Protocols are described below, and the results obtained are presented in table 1.

### Inhibition of forskolin-induced [³H]-cAMP accumulation

Human dopamine D_{2,L} receptors were cloned in fibroblast cell line CHO-K1 cells and obtained from Dr. Grandy, Vollum Institute, Portland, Oregon, USA. CHO cells were grown in a Dulbecco's modified Eagle's medium (DMEM) culture medium, supplemented with 10% heat-inactivated fetal calf serum, 2 mM glutamine, 1 mM pyruvate, 5000 units/ml penicillin, 5000 µg/ml streptomycin and 200 µg/ml at 37 °C in 93% air/7% CO₂. For incubation with test compounds, confluent cultures grown in 24 wells plates were used. Each condition or substance was routinely tested in quadruplicate. Cells were loaded with 1 µCi [³H]-adenine in 0.5 ml medium/well. After 2 hours, cultures were washed with 0.5 ml PBS containing 1 mM of the phosphodiesterase inhibitor isobutylmethylxanthine (IBMX) and incubated for 20 min with 0.5 ml PBS containing 1 mM IBMX and forskolin with or without test compound. After aspiration the reaction was stopped with 1 ml trichloroacetic acid 5% (w/v). The [³H]-ATP and [³H]-CAMP formed in the cellular extract were assayed as described by Solomon Y, Landos C, Rodbell M, 1974, A highly selective adenylyl cyclase assay, Anal Biochem 58:541-548 and Weiss S, Sebben M, Bockaert JJ, 1985, Corticotropin-peptide regulation of intracellular cyclic AMP production in cortical neurons in primary culture, J Neurochem 45:869-874. 0.8 ml Extract was passed over Dowex (50WX-4 200-400 mesh) and aluminumoxide columns, eluted with water and 0.1M imidazole (pH=7.5). Eluates were mixed with 7 ml Insta-gel and radioactivity was counted with a liquid scintillation counter. The conversion of [³H]-ATP into [³H]-cAMP was expressed as the ratio in percentage radioactivity in the cAMP fraction as compared to combined radioactivity in both cAMP and ATP fractions, and basal activity was subtracted to correct for spontaneous activity.
Reference and test compounds were all obtained as 10 mM stock solutions in 100% DMSO, and diluted in PBS/IBMX to final concentrations. Typically, compounds were used in concentrations that ranged from 10⁻¹⁰M to 10⁻⁵M. From quadruplicate data counts, the mean was taken as an estimate for drug-induced, receptor-mediated effects at specified second messenger accumulation, expressed as percentage of control values (forskolin-stimulated cAMP accumulation, subtracted by basal activity). By using the non-linear curve-fitting program INPLOT or the Excel-add-in XL-Fit, mean values were plotted against drug concentration (in molar) and a sigmoid curve (four-parameter logistic curve) was constructed. The maximal forskolin-induced stimulated conversion is taken as maximum value and the maximal inhibition (usually at drug concentrations 10⁻⁶ M or 10⁻⁵ M) as minimum and these values were fixed during the fitting process. Thus, concentrations of the compound, causing 50% of the maximally obtained inhibition of forskolin-induced cAMP accumulation (EC₅₀), are averaged over several experiments and presented as mean pEC₅₀ ± SEM in graphs and tables. Antagonist potency is assessed by co-incubating cells with a fixed agonist concentration and specified antagonist concentrations. Curve fitting procedures are identical to those used for estimating EC₅₀ values. Thus IC₅₀ values, i.e. that concentration that is able to achieves 50% of maximal antagonism that can be achieved by this compound. IC₅₀ values are corrected using a Cheng-Prussoff equation, correcting it for agonist concentration and EC₅₀ values that is obtained in the same experiment. Thus, K_{b} = IC₅₀ / (1+ [agonist]/EC₅₀, agonist).The corresponding pA₂ value is -log (K_{b}). Concentration-response curve fitting allows estimation of pEC₅₀ values and of maximal achievable effect (intrinsic activity or efficacy (ε). A full receptor agonist has ε = 1, a full receptor antagonist has ε = 0, and a partial receptor agonist has an intermediate intrinsic activity. Compound selection of partial dopamine D₂ receptor agonists therefore is completely dependent on concentration-response relationships as measured by cAMP accumulation in CHO-D_{2,L} cells and evaluation of ε, with a desired range between 0,20 and 0,60.

Several compounds, turn out to only partially inhibit formation of cAMP, e.g. terguride, preclamol ((S)-(-)-3-PPP) and SLV308. These compounds have been tested at CHO cells, stably expressing human dopamine D₂ receptors in a concentration-dependent manner and none of these compounds were able to attenuate cAMP formation by more than 60% as compared to quinpirole (100%). Thus, these compounds are identified as partial agonists. That SLV 308 is a truly partial agonist was found by applying SLV 308 itself at the dopamine receptors or in presence of the full agonist quinpirole. Thus, whereas SLV 308 is able to induce effects (inhibition of cAMP formation), it can also block the actions of a full agonist in a concentration-dependent manner (pEC₅₀8.0; pA₂ 8.4). In figure 2 the effects of SLV 308 and other reference compounds at human dopamine D₂ receptors are shown. The upper panel illustrates the agonist properties of compounds: thus, quinpirole and talipexole are full agonists, whereas SLV 308 and terguride are partial agonists. The lower panel illustrates antagonist effects against the reference agonist quinpirole. Thus, whereas haloperidol is a full antagonist at D₂ receptors, both SLV 308 and terguride are found as "partial antagonists", blocking only half of the maximal biological effect. Agonism and antagonism are in equilibrium.

### In vitro functional inhibition of [³H]-serotonin reuptake

Male rats (Wistar Hsd/Cpb: WU; 175-200 g) were decapitated, the cerebral hemispheres were rapidly removed and a P2-synaptosomal fraction was prepared. Synaptosomes were pre-incubated in absence or presence of the test compound for 15 min at 37°C, in a medium containing the monoamine oxidase inhibitor pargyline (7 µM). Subsequently, the synaptosomes were exposed to [³H]-serotonin (0.2 mM final concentration) for 10 min.
[³H]-serotonine uptake was stopped by filtration with a harvester and the non-incorporated radioactivity was removed by an extensive washing programme. The filterplates with synaptosomes were dehydrated and the amount of [³H]-serotonin present was determined by Betaplate liquid scintillation counting. Inhibitory effects on the uptake of the [³H]-serotonin were expressed as pIC₅₀ value, that is the negative logarithm of the concentration at which half maximal inhibition of radiolabeled neurotransmitter uptake is achieved. pIC₅₀ values given in Table 2 are mean values of 2-9 experiments performed in duplicate. Testcompounds, 10⁻² M dissolved in DMSO, were diluted in Krebs Ringer buffer to the testconcentrations of 10⁻⁸ to 10⁻⁵ M. Further experimental details (like e.g. buffer compositions) are described by J.T. Coyle and S.H. Snyder, 1969, Catecholamine uptake by synaptosomes in homogenates of rat brain; stereospecificity in different areas, J. Pharmacol. Exp. Ther. 170, 221-231.

### In vitro functional inhibition of [³H]-noradrenalin reuptake

Male rats (Wistar Hsd/Cpb: WU; 175-200 g) were decapitated, the hypothalamus was rapidly removed and a crude synaptosomal fraction was prepared. Synaptosomes were pre-incubated in absence or presence of the test compound for 10 min at 37°C, in a medium containing the monoamine oxidase inhibitor pargyline (7 µM). Subsequently, the synaptosomes were exposed to [³H]-noradrenaline (0.4 mM final concentration) for 15 min.
[³H]- noradrenaline uptake was stopped by filtration with a harvester and the non-incorporated radioactivity was removed by an extensive washing programme. The filterplates with synaptosomes were dehydrated and the amount of [³H]-noradrenaline present was determined by Betaplate liquid scintillation counting. Inhibitory effects on the uptake of the [³H]- noradrenaline were expressed as pIC₅₀ value, that is the negative logarithm of the concentration at which half maximal inhibition of radiolabeled neurotransmitter uptake is achieved. pIC₅₀ values given in Table 2 are mean values of 2-9 experiments performed in duplicate. Testcompounds, 10⁻² M dissolved in DMSO, were diluted in Krebs Ringer buffer to the testconcentrations of 10⁻⁸ to 10⁻⁵ M. Further experimental details (like e.g. buffer compositions) are described by J.T. Coyle and S.H. Snyder, 1969, Catecholamine uptake by synaptosomes in homogenates of rat brain; stereospecificity in different areas, J. Pharmacol. Exp. Ther. 170, 221-231.

**Table 1:**

| ***In vitro* functional activity at cloned human dopamine D**_{**2,L**} **receptors as measured by accumulation of radiolabeled cAMP (potency: pEC**_{**50**}**, intrinsic activity ε) and *in vitro* functional activity on serotonin and noradrenaline reuptake sites of pure compounds and combination preparations.** | | | | | |
|---|---|---|---|---|---|
| | | **CAMP accum.** | | **5-HT**_{**uptake**} | **NA**_{**uptake**} |
| **Compound class** | **Compound** | **pEC**_{**50**}***** | **ε*** | **pIC**_{**50**} | **pIC**_{**50**} |
| | | | | | |
| Full dopamine-D₂ agonist | quinpirole | **7.0** | **1.00** | **< 5.0** | **< 5.0** |
| Full dopamine-D₂ agonist | talipexole | **7.4** | **1.00** | **< 5.0** | **< 5.0** |
| | | | | | |
| Partial dopamine-D₂ agonist | terguride | **9.4** | **0.52** | | |
| Partial dopamine-D₂ agonist | preclamol | **6.4** | **0.36** | **< 5.0** | **5.3** |
| Partial dopamine-D₂ agonist | bifeprunox | **7.8** | **0.20** | **4.8** | **4.6** |
| Partial dopamine-D₂ agonist | SLV 308 | **7.5** | **0.55** | **< 5.0** | **< 5.0** |
| | | | | | |
| Specific 5-HT reuptake inh. | fluvoxamine | | | **6.5** | **4.9** |
| Specific 5-HT reuptake inh. | fluoxetine | | | **5.9** | **5.0** |
| Specific 5-HT reuptake inh. | paroxetine | | | **7.8** | |
| | | | | | |
| Specific NA reuptake inh. | DMI | | | **5.0** | **7.5** |
| Specific NA reuptake inh. | reboxetine | | | **5.0** | **7.2** |
| | | | | | |
| Partial D₂ agonist + SRI | example **1a** | | **0.35** | | |
| Partial D₂ agonist + SRI | example **1b** | | **0.36** | | |
| Partial D₂ agonist + SRI | example **1c** | **6.9** | **0.59** | **7.9** | **5.0** |
| Partial D₂ agonist + SRI | example **1d** | | **0.42** | **6.4** | |
| Partial D₂ agonist + SRI | example **1e** | **> 9.0** | **0.60** | **6.4** | **< 5.0** |
| ****PEC***_{***50***}***:*** *-log of the concentration at which half of the maximally achievable effect is obtained for that particular drug. Its intrinsic activity, *ε, is expressed as a fraction of full agonism (*ε=*1), which is achieved by a full agonist such as quinpirole.* | | | | | |

Microdialysis allows an insight into changes in neurotransmitters and their metabolites in the brain extracellular space in discrete brain regions in awake freely moving animals. Extracellular levels of neurotransmitters mirror the neuronal activity (neurotransmitter release) in these brain regions and can be influenced by selective receptor agonists and antagonists, uptake inhibitors, etc. The in vivo effects of the (mixtures of) compounds of the invention on dopamine, serotonin and noradrenalin levels were determined by microdialysis according to the protocol given below:

### Dopamine and serotonin measurements by in vivo microdialysis

**Surgery.** Male wistar rats, weighing 280-300g, were anaesthetized by halothane-narcosis (1.5% halothane in NO₂/O₂ 2:1). Antibacterial agents and analgesics were administered prior to (Baytril (150 µl/rat i.m.)) and after (Temgesic (0.005-0.01 mg/kg i.m. non-diluted/rat)) surgery. Following placement in a stereotactic frame, the skull is exposed and a 1mm bore-hole is drilled through the bone above the nucleus accumbens (co-ordinates from interaural point (mm): anterior-posterior +10.5, mediolateral -2.1 and dorsoventral -6.5 at a 8° angle (from dura)) in those animals where determination of dialysate dopamine and serotonin levels is required. In other animals, the skull is exposed and a 1mm bore-hole is drilled through the bone above the prefrontal cortex (co-ordinates from bregma (mm): anterior-posterior +3.2, mediolateral -0.6 and dorsoventral -1.5 at a 0° angle (from dura)) in those animals where determination of dialysate noradrenaline levels is required. Smaller bore holes are made and three screws are inserted within the skull. The intracerebral guide cannula (CMA, Carnegie) is lowered through the hole until the tip is immediately above the nucleus accumbens or prefrontal cortex. The guide together with the screws are cemented onto the bone with dental cement and the surrounding skin sutured. Animals are allowed to recover at least six days prior to microdialysis experimentation.

**Microdialysis experimentation**. On the day of the experiment microdialysis probes (CMA 12, 0.5mm outer diameter, Stockholm, Sweden) are inserted through the guide cannula into the nucleus accumbens (2mm membrane length) or prefrontal cortex (4mm membrane length). The inlet of the probe is connected with low volume tubing (F.E.B- tubing, 1.2 µl/10 cm, Camegie) via a liquid-swivel (dual channel; Instech, UK) on a counterbalanced arm, to a syringe-pump (Harvard, 10 channel). The syringe pump delivers the dialysis fluid (147mM NaCI, 4mM KCI, 1.2mM CaCl₂ and 0.7mM MgCl₂) at a constant flow of 2µl/min. The outlet of the probe is connected with low volume tubing via the liquid swivel to a CMA 140 fraction collector. The tubing is supported by a stainless steel wire which leads from the swivel down to a clip, which fits to a collar around the neck of an animal. Dopamine, serotonin and noradrenaline levels are stable 16 hours after probe insertion, after which dialysis sampling begins. Samples are collected at a flow rate of 2µl/min at 20min intervals (40µl volume) in vials containing 50µl of a HCOOH/cysteine solution (0.02M/ 0.2 w/v%) to prevent oxidation of the compounds. Following a baseline period of 5 samples, drugs are administered systemically and at least a further 8 samples collected. All samples were stored post-collection on dry ice and frozen at -80°C prior to analysis by high-performance liquid chromatography (HPLC) coupled to electrochemical detection described below.

**Analysis of dialysate dopamine and serotonin**. Samples are analysed using a reversed phase column (Supelcosyl LC-8DB, 25 cm x 4.6 mm, 5µm particle diameter, Supelco), maintained at 45°C with a column-oven (Mistral; Spark, The Netherlands), and a Gilson (model 231-401 or 232-401) or HP1100 auto-injector with cooling device (10°C). The pump (Hewlett Packard, model 1050 or HP1100) operates at a flow of 1 ml/min. The mobile phase consisted of (mM) 50 HAc/NaAc (3:1), 1.46 HSA, 0.27 EDTA and 16% (v/v) methanol. The final pH was adjusted to 4.9 with 1N NaOH. Dopamine and serotonin are electrochemically detected with an EG&G (model 400, Princeton Applied Research) controller equipped with a glassy carbon working electrode (VT-03; Antec, Leiden, the Netherlands). The potential is set at 600 mV versus an Ag/AgCl reference electrode. The output is recorded on a computer equipped with Hyperchem™ (Hewlett-Packard Inc.) which measures peak height values. Calculations (pg /20 min) are made using peak height values of analysed standard solutions containing known amounts of dopamine and serotonin. Analysis of dialysate dopamine and noradrenaline. The samples are analysed using a reversed phase column (Supelcosyl LC-18DB, 150 mm x 4.6 mm, dp = 3 µm, Supelco), maintained at 25°C with a column-oven (Mistral; Spark, The Netherlands), and a Gilson (model 231-401 or 232-401) or HP1100 autoinjector with cooling device (10°C). The pump (Hewlett Packard, model 1050 or HP1100) operates at a flow of 1 ml/mm. The basal mobile phase consists of: 50 mM NaAc, and 0.27 mM EDTA. The final concentration of 1-octanesulfonic acid (NOS) and methanol, as well as the final pH (adjusted with HAc), vary with the different brain areas under examination. The compounds are electrochemically detected with an EG&G (model 400, Princeton Applied Research) controller equipped with a glassy carbon working electrode (VT-03; Antec, Leiden, the Netherlands). The potential is set at 450 mV versus a Hyref reference electrode. The output is analyzed and archived with an Hewlett Packard Chemstation which calculates concentration (pg/20 min) on peak height values. Calculations are made using peak height values of analysed standard solutions containing known amounts of the compounds (external standard method). The results obtained are given in table 2:

**Table 2:**

| *In vivo* microdialysis data on dopamine, serotonin and noradrenaline levels. | | | | |
|---|---|---|---|---|
| **Compound class Compound** | | **[dopamine]** | **[serotonin]** | **[noradren]** |
| | | **ED**_{**75**} **mg/kg** | **ED**_{**150**} **mg/kg** | **ED**_{**150**} **mg/kg** |
| | | | | |
| Full dopamine-D₂ agonist | quinpirole | *0.04* | *> 3* | |
| Full dopamine-D₂ agonist | talipexole | **< 0.1** | **>10** | |
| | | | | |
| partial dopamine-D₂ agonist | preclamol | | *>30* | |
| partial dopamine-D₂ agonist | bifeprunox | *>10* | *>10* ^{*1*} | |
| partial dopamine-D₂ agonist | SLV 308 | *0.04* | *0.45* ^{*1*} | |
| | | | | |
| Specific 5-HT reuptake inh. | fluvoxamine | **>30** ^{**2**} | **1.28** | |
| Specific 5-HT reuptake inh | paroxetine | **>10** | **< 10** | |
| | | | | |
| Specific NA reuptake inhib. | DMI | **> 3** | **> 3** | **1.64** |
| Specific NA reuptake inhib. | reboxetine | **> 3** | **> 3** | |
| | | | | |
| Partial D₂ agonist + SRI | example **1c** | **3.92** | **14.79** | |
| *Table* 2. *Effect of full and partial D*_{*2*} *agonists alone and in combination with either a serotonin reuptake inhibitor (SRI) or noradrenaline reuptake inhibitor (NRI) on dialysate dopamine and serotonin in the nucleus* accumbens *and noradrenaline levels in the prefrontal* cortex *of the awake freely moving rat. Values in **bold** are p.o., values in italic are i.p.* ^{*1*}*: ED*_{*75*}*,* ^{*2*}*: ED*_{*150*}. | | | | |

## Claims

1. Use of a compound or a combination of compounds having partial dopamine-D₂ receptor agonistic activity and serotonin and/or noradrenaline reuptake inhibitory activity for the preparation of a pharmaceutical composition for the treatment of disorders caused by disturbances of the dopamine, serotonin and/or noradrenaline systems or that can be treated via manipulation of those systems.

2. Use as claimed in claim 1 **characterized in that** said compound or combination of compounds have partial dopamine-D₂ receptor agonistic activity and serotonin reuptake inhibitory activity, for the preparation of a pharmaceutical composition for the treatment of disorders caused by disturbances of the dopamine and serotonin systems or that can be treated via manipulation of those systems.

3. Use as claimed in claim 1 **characterized in that** said compound or combination of compounds have partial dopamine-D₂ receptor agonistic activity and noradrenergic reuptake inhibitory activity, for the preparation of a pharmaceutical composition for the treatment of disorders caused by disturbances of the dopamine and noradrenaline systems or that can be treated via manipulation of those systems.

4. Use as claimed in claim 1 **characterized in that** said compound has partial dopamine-D₂ receptor agonistic and serotonin and/or noradrenaline reuptake inhibitory activity combined in one molecule.

5. Use as claimed in claim 1 **characterized in that** said combination of compounds has partial dopamine-D₂ receptor agonistic activity and serotonin and/or noradrenaline reuptake inhibitory activity.

6. Use as claimed in claim 2 **characterized in that** said compound has partial dopamine-D₂ receptor agonistic and serotonin reuptake inhibitory activity combined in one molecule.

7. Use as claimed in claim 2 **characterized in that** said combination of compounds has partial dopamine-D₂ receptor agonistic activity and serotonin reuptake inhibitory activity

8. Use as claimed in claim 3 **characterized in that** said compound has partial dopamine-D₂ receptor agonistic and noradrenaline reuptake inhibitory activity combined in one molecule.

9. Use as claimed in claim 3 **characterized in that** said combination of compounds has partial dopamine-D₂ receptor agonistic activity and noradrenaline reuptake inhibitory activity.

10. Use as claimed in claims 1-9 **characterized in that** said composition is used for the treatment of psychiatric and/or neurologic disorders caused by disturbances of the major monoaminergic (dopamine, serotonin and/or nordrenaline) systems or that can be treated via manipulation of those systems, said disorders selected from the group consisting of: schizophrenia and other psychotic disorders; mood disorders such as bipolar I disorders, bipolar II disorders and unipolar depressive disorders like minor depression, seasonal affective disorder, postnatal depression, dysthymia and major depression; anxiety disorders including panic disorder (with or without agoraphobia), social phobia, obsessive compulsive disorder (OCD, with or without co-morbid chronic tic or schizotypal disorder), posttraumatic stress disorder and generalized anxiety disorder (GAD); substance related disorders, including substance use disorders (like dependence and abuse) and substance induced disorders (like substance withdrawal); pervasive development disorders including autistic disorder and Rett's disorder; attention deficit and disruptive behavior disorders such as attention deficit hyperactivity disorder (ADHD); impulse control disorders like pathological gambling; eating disorders like anorexia nervosa and bulimia nervosa; tic disorders like Tourette's disorder; restless legs syndrome; disorders **characterized by** impairment of cognition, memory and/or co-morbid psychiatric disorders and neurorehabilitation (post traumatic brain lesions).

11. Use as claimed in claims 1-9 **characterized in that** said composition is used for the treatment of schizophrenia and other psychotic disorders.

12. Use as claimed in claims 1-9 **characterized in that** said composition is used for the treatment of mood disorders such as bipolar I disorders and bipolar II disorders.

13. Use as claimed in claims 1-9 **characterized in that** said composition is used for the treatment of unipolar depressive disorders like minor depression, seasonal affective disorder, postnatal depression, dysthymia and major depression.

14. Use as claimed in claims 1-9 **characterized in that** said composition is used for the treatment of anxiety disorders including panic disorder (with or without agoraphobia).

15. Use as claimed in claims 1-9 **characterized in that** said composition is used for the treatment of social phobia.

16. Use as claimed in claims 1-9 **characterized in that** said composition is used for the treatment of obsessive compulsive disorder (OCD, with or without co-morbid chronic tic or schizotypal disorder).

17. Use as claimed in claims 1-9 **characterized in that** said composition is used for the treatment of posttraumatic stress disorder.

18. Use as claimed in claims 1-9 **characterized in that** said composition is used for the treatment of generalized anxiety disorder (GAD).

19. Use as claimed in claims 1-9 **characterized in that** said composition is used for the treatment of substance related disorders, including substance use disorders (like dependence and abuse) and substance induced disorders (like substance withdrawal).

20. Use as claimed in claims 1-9 **characterized in that** said composition is used for the treatment of pervasive development disorders including autistic disorder and Rett's disorder

21. Use as claimed in claims 1-9 **characterized in that** said composition is used for the treatment of attention deficit and disruptive behavior disorders such as attention deficit hyperactivity disorder (ADHD).

22. Use as claimed in claims 1-9 **characterized in that** said composition is used for the treatment of impulse control disorders like pathological gambling.

23. Use as claimed in claims 1-9 **characterized in that** said composition is used for the treatment of eating disorders like anorexia nervosa and bulimia nervosa.

24. Use as claimed in claims 1-9 **characterized in that** said composition is used for the treatment of tic disorders like Tourette's disorder.

25. Use as claimed in claims 1-9 **characterized in that** said composition is used for the treatment of restless legs syndrome.

26. Use as claimed in claims 1-9 **characterized in that** said composition is used for the treatment of disorders **characterized by** impairment of cognition, memory and/or co-morbid psychiatric disorders and neurorehabilitation (post brain lesions).

27. Method for the preparation of a composition **characterized in that** it brings a compound having partial dopamine-D₂ receptor agonistic activity and serotonin and/or noradrenaline reuptake inhibitory activity, or a combination of compounds having partial dopamine-D₂ receptor agonistic activity and serotonin and/or noradrenaline reuptake inhibitory activity, into a form suitable for administration.

28. A composition comprising a compound having partial dopamine-D₂ receptor agonistic activity and serotonin and/or noradrenaline reuptake inhibitory activity, or a combination of compounds having partial dopamine-D₂ receptor agonistic activity and serotonin and/or noradrenaline reuptake inhibitory activity, in a form suitable for administration.

29. Use as claimed in claims 1-26 **characterized in that** said partial dopamine-D₂ agonistic activity intrinsically is between 20% and 60% of that of a full agonist in the inhibition of forskolin-induced [³H]-cAMP accumulation.

30. Use as claimed in claims 1-28 **characterized in that** said composition simultaneously shows partial dopamine-D₂ activity and serotonin and/or noradrenaline reuptake inhibitory activity in in vivo microdialysis experiments.

31. Use as claimed in claims 1-28 **characterized in that** said composition simultaneously shows partial dopamine-D₂ activity and serotonin reuptake inhibitory activity in in vivo microdialysis experiments.

32. Use as claimed in claims 1-28 **characterized in that** said composition simultaneously shows partial dopamine-D₂ activity and noradrenaline reuptake inhibitory activity in in vivo microdialysis experiments.
